# EUROPÄISCHE PATENTSCHRIFT

(11) **EP 0 458 241 B1**
(45) Veröffentlichungstag und Bekanntmachung des Hinweises auf die Patenterteilung: **07.12.1994**
(21) Anmeldenummer: 91108141.2
(22) Anmeldetag: 21.05.1991
(51) Int. Cl.: B01D 61/24, C12M 1/12, C12M 3/06

(54) **Verfahren und Vorrichtung zur Abtrennung von Ammonium aus wässrigen Flüssigkeiten**
Process and apparatus for separating ammonium from aqueous liquids
Procédé et appareil pour la séparation d'ammonium à partir de liquides aqueux

(30) Priorität: 25.05.1990 DE 4016971
(43) Veröffentlichungstag der Anmeldung: 27.11.1991
(73) Patentinhaber: FORSCHUNGSZENTRUM JÜLICH GMBH, 52425 Jülich (DE)
(72) Erfinder: Biselli, Manfred, W-5170 Jülich (DE); Thömmes, Jörg, W-5300 Bonn 1 (DE); Wandrey, Christian, Prof., W-5170 Jülich (DE)

(56) Entgegenhaltungen:
- DECHEMA BIOTECHNOLOGY CONFERENCES 3 - VCH VERLAGSGES., 1989, Seiten 567-570; T.PULTAR et al.: "Fermentation optimization of lysolipin X by continuous ammonium extraction"
- P.B. SOETERS et al.: "Advances in Ammonia Metabolism and Hepatic Encephalopathy",Chapter 69, 1988, Seiten 534-642,Elsevier Science Publishers B.V. (Biomedical Division); M.BUSSE et al.: "Detoxification of ammonia utilizing a lipophilic hollow-fiber reactor"
- DATABASE WPIL, Woche 8740, AN=87-280987 [40], Derwent Publications Ltd, London,GB; & JP-A-62 195 351 (AJINOMOTO K.K.) 28-08-1987,& PATENT ABSTRACTS OF JAPAN, Band 12, Nr. 47 (C-475)
- DATABASE WPIL, Woche 8734, AN=87-238114 [34], Derwent Publications Ltd, London,GB; JP-A-62 160 189 (AJINOMOTO K.K.) 16-07-1987,& PATENT ABSTRACTS OF JAPAN, Band 11, Nr. 398 (C-466)
- CHEMICAL ABSTRACTS, Band 84, Nr. 22, 31. Mai 1976, Seiten 322-323,Zusammenfassung Nr. 155197c, Columbus, Ohio, US; J.L. EISENMANN et al.:"Selective nutrient removal from secondary effluent"

## Beschreibung

Die Erfindung bezieht sich auf ein Verfahren zur Abtrennung von Ammonium aus kationenhaltigen wäßrigen Flüssigkeiten, insbesondere Fermentationsbrühen, über eine Transportmembran und selektive Elimination auf der Akzeptorseite sowie auf eine dafür geeignete Vorrichtung.

Ammoniumionen wirken bei vielen Fermentationsprozessen inhibierend auf das Wachstum und die Produktivität. Das spielt insbesondere eine Rolle bei der Kultivierung von Säugerzellen. Es ist daher zweckmäßig, die Ammoniumkonzentration in Fermentationsbrühen abzusenken, um höhere Biomassekonzentrationen sowie höhere Produktivitäten zu erreichen.

Es wurde bereits ein Verfahren zur Verminderung der Ammoniumkonzentration solcher Flüssigkeiten entwickelt, das auf dem Gleichgewicht zwischen Ammonium und Ammoniak solcher Kulturflüssigkeiten basiert, das in wenigen Prozenten darin enthalten ist (wissenschaftlicher Ergebnisbericht der GBF 1985, Seiten 20 - 22): Danach wird das Ammoniak über eine poröse Membran (Polypropylenschlauch) durch Diffusion abgetrennt und auf der Sekundärseite der Membran durch ein saures Medium aufgenommen und die dabei gebildeten Ammoniumionen durch Ionenaustausch entfernt. Über diese Verfahrensweise wird auch von Soeters et al. in "Advances in Ammonia Metabolism and Hepatic Encephalopathy" 69 (1988) 534 - 42 berichtet.

Von Busse et al. wird in J. Hepatology 1 to Vol. 4 (1987) S. 10 die Entfernung von Ammonium mittels des Lipid Hollowfiber Membrane Reactors beschrieben. Hier diffundieren donorseitige Ammoniumionen als Ammoniak durch eine lipophile Hollowfibermembran und werden akzeptorseitig entweder wie zuvor durch einen pH-shift, oder enzymatisch mit α-Ketoglutarat und NADH zu Glutaminsäure umgesetzt und somit dem Transportgleichgewicht entzogen.

T. Pultar et al., Dechema Biotechnolgy Conferences 3 - VCH Verlagsgesellschaft 1989, S. 567 - 71, erwähnen die Entfernung von Ammoniumionen aus Kulturflüssigkeiten durch Anlagerung der Ammoniumionen an Calciumphosphat, die jedoch als mangelhaft bezeichnet wird, weshalb die vorstehend skizzierte Ammoniakdiffusion durch eine poröse Membran, deren Akzeptorseite mit saurer Lösung gespült wird, favorisiert werden soll.

Ein anderes Verfahren zur Absenkung der Ammoniumkonzentration wird von M. Iio et al. in R. Murakami (Herausg.) "Growth and Differenciation of Cells in Defined Environment" Springer-Verlag, New York 1984, S. 437 - 42, angegeben: hier wird die Absorption von Ammonium durch eine Suspension von hydrothermal erzeugtem Aluminiumsilicat ZCP-50 in einem Dialyseröhrchen ausgenutzt.

Von T.E. Hassell et al. in Spier und Griffiths (Herausg.) "Modern Approaches to Animal Cell Technology" Butterworths, London 1987, S. 245 -63, wird auf die Ammoniumminderung durch Ersatz von Glutamin durch Glutamat oder 2-Oxo-glutarat hingewiesen.

Die vorstehend genannten Techniken zur Verminderung der Ammoniumkonzentration von Zellkulturflüssigkeiten erscheinen nicht voll befriedigend, insbesondere kann die weitgehend favorisierte Abtrennung durch Ammoniakdiffusion über längere Zeiten hinweg infolge einer Veränderung oder Verstopfung der Porenstruktur Probleme bereiten. Ferner sind die erreichbaren Transportgeschwindigkeiten durch die Membran gering, so daß sehr große Membranflächen benötigt werden.

Ziel der Erfindung ist daher ein problemlos durchführbares, leistungsfähiges Verfahren zur Absenkung des Ammoniumgehalts in wäßrigen Flüssigkeiten, insb. Zellflüssigkeiten, mit dem längere Betriebszyklen und höhere Stoffaustauschleistungen erzielt werden können.

Das zu diesem Zweck entwickelte erfindungsgemäße Verfahren der eingangs genannten Art ist im wesentlichen dadurch gekennzeichnet,
daß man als Membran eine Kationenaustauschermembran verwendet und akzeptorseitig einen gegenüber der Donorseite derart erhöhten pH-Wert vorsieht, daß quer zur Membran ein NH₄⁺-Konzentrationsgefälle aufgrund des Gleichgewichts zwischen Ammonium und Ammoniak vorliegt, welch letzteres aus der akzeptorseitigen Lösung entfernt wird, deren Gehalt an von NH₄⁺ verschiedenen Kationen demjenigen der donorseitigen Lösung entspricht und/oder deren am Austausch über die Membran beteiligtes Volumen höchstens 10 % des donorseitigen Gesamtflüssigkeitsvolumens ausmacht.

Weitere Besonderheiten gehen aus den abhängigen Ansprüchen hervor.

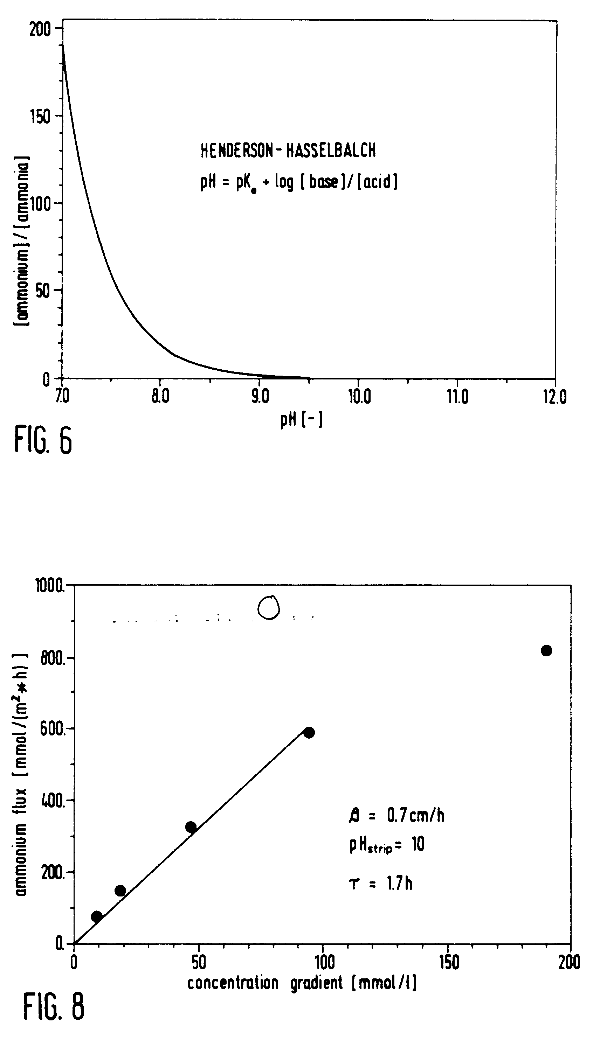

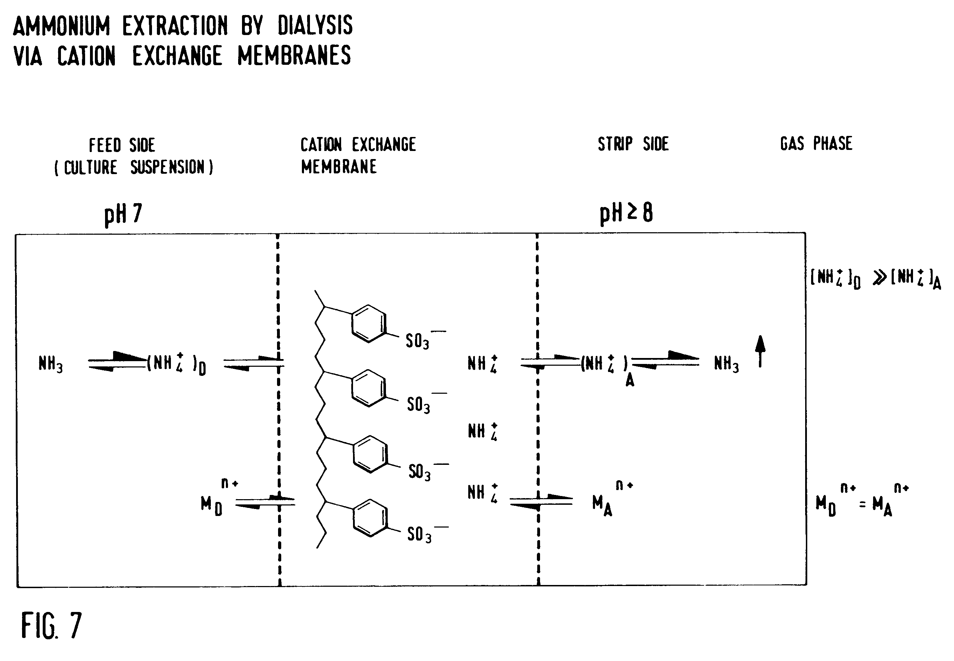

Erfindungsgemäß wird also die Selektivität der Ammoniumabtrennung nicht, wie bisher üblich, durch NH₃-Diffusion durch eine hydrophobe Membran erreicht, sondern durch Angleichung der Kationengehalte auf Donor- und Akzeptorseite und ein starkes NH₄⁺ -gefälle zur Akzeptorseite hin, das den Transport von NH₄⁺ durch eine Kationenaustauschermembran fördert. Dabei wird die in biologischen Systemen mit pH-Werten um den Neutralpunkt im Vergleich zul NH₃-Konzentration sehr viel höhere NH₄⁺-Konzentration(siehe Fig. 6) als "Motor" für einen verstärkten Ammoniumtransport zur Akzeptorseite ausgenutzt, wo durch einen erhöhten pH-Wert für eine niedrige NH₄⁺-Konzentration durch gleichgewichtsbedingte Deprotonierung zum NH₃ gesorgt wird. Diese diffusionsfördernde NH₄⁺ - Erniedrigung auf der Akzeptorseite wird zusätzlich durch NH₃-Entfernung aus dem akzeptorseitigen System verstärkt (siehe Fig. 7). Die mit steigendendem Konzentrationsgradienten quer zur Membran zunehmende NH₄⁺-Transportrate geht aus Fig. 8 hervor.

Die Ammoniakentfernung aus der akzeptorseitigen Lösung kann u.a. durch Unterdruck oder Hindurchleiten von Gas (Variante A) erfolgen, insbesondere wird jedoch von der an sich bekannten Methode der Diffusion von Ammoniak durch eine poröse Membran mit sekundärseitiger Protonierung zu Ammonium Gebrauch gemacht (Variante B). Global laufen folgende Schritte ab: 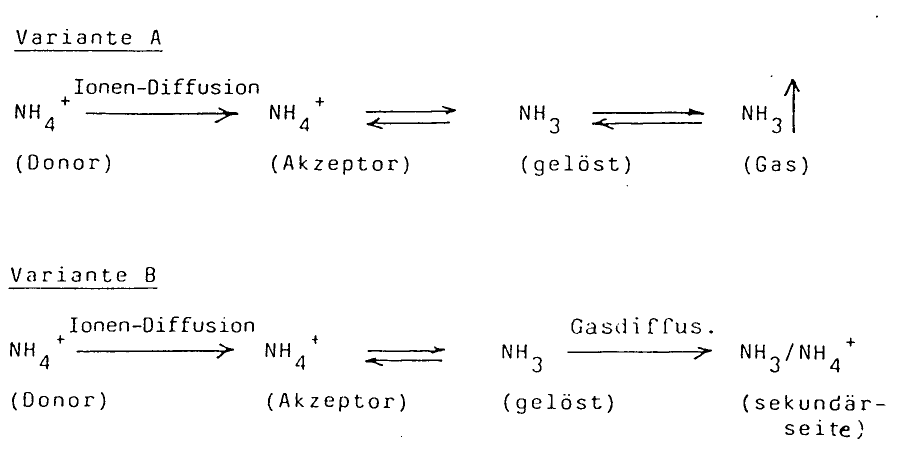
Grundsätzlich kann mit stagnierender Akzeptorlösung gearbeitet werden, aus der das Ammoniak fortlaufend insbesondere durch Anlegen von Unterdruck oder Durchleiten von Inertgas entfernt wird, vorzugsweise wird jedoch akzeptorseitig kontinuierlich ein Strom von Akzeptorlösung über die Membran und dann zu einer Anordnung zur NH₃-Entfernung geleitet.

In einer realen Fermentationsbrühe sind neben Ammoniak zahlreiche andere Kationen vorhanden, deren Transport durch die begrenzende Kationenaustauschermembran mehr oder minder gleichberechtigt zum Ammonium erfolgt. Dabei passieren kleinere Ionen die Membran aufgrund ihrer höheren Beweglichkeit besonders schnell. Um merkliche Kationenverluste und damit eine Konzentrationsminderung derselben in der Donorlösung zu vermeiden, sind unterschiedliche Verfahrensweisen möglich, insbesondere kann in folgender Weise verfahren werden:
Als Akzeptorlösung kann ein zellfreies, aber mit allen zum Wachstum notwendigen kationischen Inhaltsstoffen versehenes Fermentationsmedium (aber mit erhöhtem pH-Wert) dienen, das alle erforderlichen Kationen in gleicher Konzentration enthält, deren Ausdünnung auf der Donorseite somit vermieden wird.

Die für das Wachstum der Biomasse in der Fermentations- d.h. Donorlösung essentiellen Kationen befinden sich also auf beiden Seiten in Gleichgewichtskonzentration, ein Auswandern aus der Fermentationsbrühe erfolgt nicht. Da sich in der Akzeptorlösung aber kein biologisches System befindet, entsteht kein Ammonium, d.h. der zur Entfernung des Ammoniums aus der Donorlösung erforderliche Konzentrationsgradient ist vorhanden. Diese Akzeptorlösung kann kontinuierlich an der Membran vorbeigeführt werden und somit ständig donorseitig gebildetes und die Membran passierendes Ammonium aufgrund des pH-Sprungs als Ammoniak aufnehmen.

Alternativ oder zusätzlich kann die Akzeptorlösung im Verhältnis zur Donorlösung ein kleines Volumen haben und nicht kontinuierlich ausgetauscht werden. Auf diese Art und Weise stellt sich aufgrund des geringen Volumens schnell eine Gleichgewichtskonzentration zwischen Donor- und Akzeptorlösung ein, deren Wert nahe am ursprünglichen Wert der Donorlösung liegt, d.h. bei der wenig Kationen aus der Donorlösung zur Einstellung des Gleichgewichts abgezogen werden. Die Konzentration an Ammonium erreicht dann ebenfalls schnell einen Gleichgewichtswert, der durch die Löslichkeit des durch Deprotonierung entstehenden Ammoniaks in der Akzeptorlösung bestimmt ist. Der Ammoniak muß daher aus der Akzeptorlösung entfernt werden, um den zum Transport erforderlichen Konzentrationsgradienten aufrechtzuerhalten.

Hierzu kann Helium durch die Akzeptorlösung geleitet werden, um diese zu entgasen. Es bietet sich auch das Anlegen eines leichten Unterdrucks zu diesem Zweck an. Wichtig ist ein ständiges Entfernen des gelösten Ammoniaks aus der Akzeptorlösung in die Gasphase über der Lösung, um einen kontinuierlichen Transport von Ammoniumionen durch die Membran entlang des Konzentgrationsgradienten von Donor- zur Akzeptorlösung zu ermöglichen. Dieser Gradient bleibt dann durch die Weiterreaktion des Ammoniums zu Ammoniak in der alkalischen Akzeptorlösung bestehen.

Besonders zweckmäßig wird jedoch Ammoniak durch Diffusion durch eine poröse Membran abgetrennt, wie bereits oben erwähnt, die Begrenzung für eine stagnierende Akzeptorlösung sein kann, vorzugsweise wird jedoch ein Kreislauf von Akzeptorlösung vorgesehen, der sowohl über die Akzeptorseite der Kationentauschermembran als auch über die Primärseite der porösen NH₃-Diffusionsmembran geleitet wird, deren Sekundärseite von einem Strom von saurer Protonierungslösung umspült wird, die ohne weiteres verworfen werden kann.

Eine zur Durchführung des Verfahrens geeignete Vorrichtung zur Ammonium-Abtrennung aus kationenhaltiger wäßriger Flüssigkeit mit einem Behälter die kationenhaltige Flüssigkeit enthält und in eine Zirkulationsleitung (Bypaß) mit einer einkuppelbaren Durchflußstrecke einbezogen ist, ist dadurch gekennzeichnet, daß die Durchflußstrecke eine Kationenaustauschermembran als eine Begrenzungswand aufweist, die gleichzeitig Begrenzungswand eines Akzeptorraumes ist, der entweder unmittelbar Einrichtungen zur pH-Regelung und NH₃-Entfernung enthält oder Teil einer solche Einrichtungen enthaltenden Zirkulationsleitung ist.

Nachfolgend wird die Erfindung anhand eines Beispiels unter Bezugnahme auf die angefügten Zeichnungen beschrieben. Es zeigen:
- Figuren 1 und 2: je ein Schema für eine erfindungsgemäße Anordnung;
- Figur 3: ein Kurvenbild für die Ammoniumkonzentration von Donor- und Akzeptorlösung Dei Anwendung des erfindungsgemäßen Verfahrens in Abhängigkeit von der Zeit;
- Figur 4: ein Diagramm für die Übergangsgeschwindigkeiten von Ammoniumionen durch die Kationenaustauschermembran in Abhängigkeit vom pH-Wert der Akzeptorseite
- Figur 5: ein Diagramm für den Ammonium-Restanteil (Gleichgewichts-Wert durch Startkonzentration) auf der Donorseite in Abhängigkeit vom Akzeptor-pH und
- Fig. 6 - 8: Kurven zur Veranschaulichung des erfindungsgemäßen NH₄⁺-Abtrennprinzips.

### Beispiel

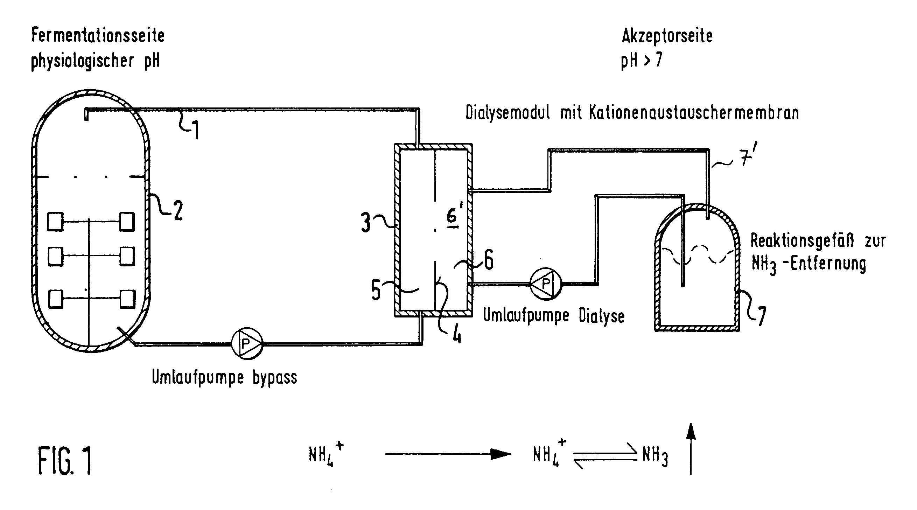

Verwendet wurde eine Dialyseapparatur gemäß Figur 1: In den Bypaß 1 eines Fermenters 2 ist ein Dialysemodul 3 eingeschaltet, in dem eine Kationenaustauschermenbran 4 (z.B. Polystyrolsulfonat "Permion 4010" der Firma SERVA; Membranfläche: 13,4 cm²) einen Donorstrom 5 von einem Akzeptorstrom 6 durch den Raum 6' trennt. Dieser Akzeptorstrom 6 wird im Kreislauf 7' über ein Reaktionsgefäß 7 geleitet, in dem Ammoniak gasförmig als NH₃ ausgetrieben wird.

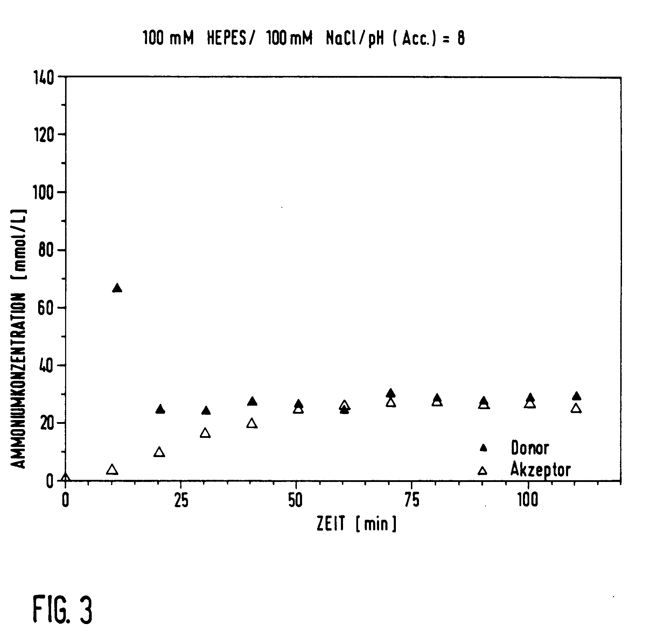

In dieser Apparatur wurde in einem Modellversuch ohne Biomasse eine wäßrige Lösung aus 100mM HEPES-Puffer (pH 7), 100 mM NaCl und 100 mM Ammoniumchlorid dornorseitig gegen eine Lösung aus 100 mM HEPES-Puffer (pH 8) und 100 mM NaCl akzeptorseitig dialysiert. Das Entgasen und somit die Entfernung des durch Deprotonierung entstehenden Ammoniaks erfolgte durch Durchperlen eines moderaten Heliumstroms durch die Akzeptorlösung. In Abständen von 10 Min. wurden donor- und akzeptorseitig Proben auf ihren Ammoniumgehalt hin untersucht (s. Fig. 3). Es ergab sich ein linearer Abfall der Ammoniunkonzentration donorseitig bis zu einem Gleichgewichtswert. Aus dem Abfall der Ammoniumkonzentration donorseitig errechnet sich die Geschwindigkeit des Stoffübergangs. Sie betrug im vorliegenden Beispiel 5.5 mol/h · m² Membranfläche.

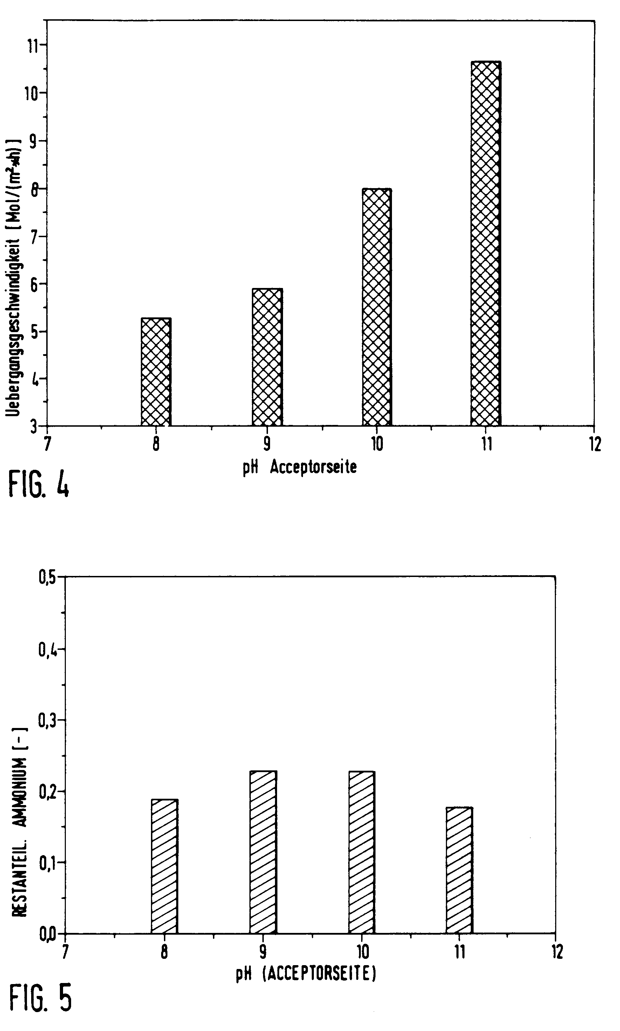

Die Untersuchung der Stoffübergangsgeschwindigkeit in Abhängigkeit vom pH der Akzeptorlösung ergab einen Anstieg derselben mit steigendem pH (s. Fig. 4). Die sich jeweils donorseitig einstellende Gleichgewichtskonzentration an Ammonium, die sich als Restanteil (Gleichgewichtskonzentration : Anfangskonzentration) ausdrücken läßt, ist offensichtlich vom pH der Akzeptorlösung unabhängig (s. Fig. 5) und wird nur durch die Löslichkeit des Ammoniaks in der Akzeptorlösung bestimmt.

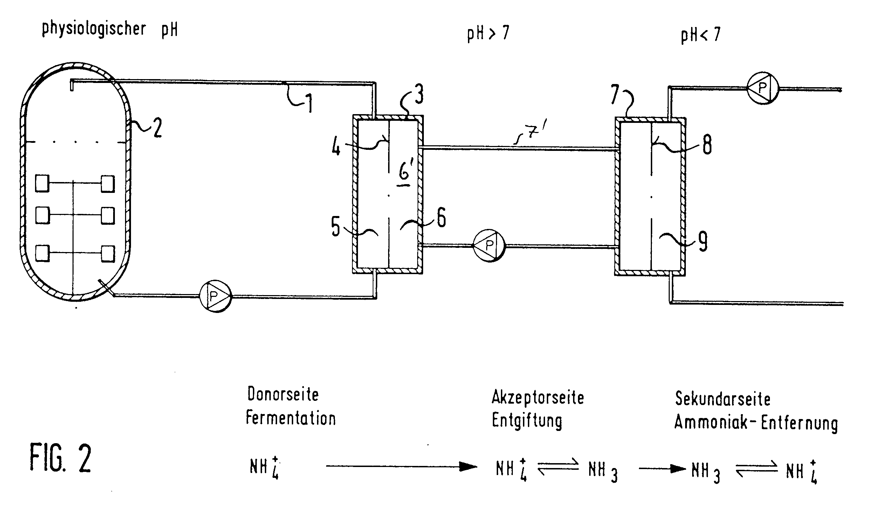

Figur 2 zeigt eine bevorzugte Variante der Dialyseapparatur aus Figur 1. In den Bypaß 1 eines Fermenters 2 ist ein Dialysemodul 3 eingeschaltet, in dem eine Kationenaustauschermembran 4 (z.B. Polystyrolsulfonat "Permion 4010" der Fa. SERVA, Membranfläche 13,4 cm²) einen Donorstrom 5 von einem Akzeptorstrom 6 trennt. Dieser Akzeptorstrom 6 wird im Kreislauf 7' durch ein zweites Dialysemodul 7 geleitet, das eine mikroporöse Membran 8 als Trenngrenze von einem Sekundärstrom enthält. Dieser (saure) Sekundärstrom 9 wird kontinuierlich an der mikroporösen Membran 8 vorbeigeleitet und nach Passieren derselben zweckmäßigerweise verworfen.

Die schematisch in zwei Varianten wiedergegebene Labor-Anordnung wurde wie folgt ausgeführt und betrieben:

### 1) Dialysemodul:

Das Dialysemodul ist zur Aufnahme einer Flachmembran konzipiert. Diese wird zwischen den beiden Modulhälften eingelegt, die Verschraubung der Modulhälften bewirkt über Silikondichtungen die Abdichtung zwischen Membran und den Modulhälften. Jede Modulhälfte besitzt zwei Anschlüsse für den Flüssigkeitszu- und ablauf. Der Flüssigkeitsstrom wird tangential mit geringem Querschnitt an der Membran vorbeigeführt, um eine hohe Überströmgeschwindigkeit zu erreichen. Die erforderliche Überströmfläche erhält man durch eine mäanderartige Stromführung. Als Werkstoff empfiehlt sich aus Gründen der Autoklavierbarkeit Edelstahl, der eine minimale Rauhigkeit an den Kontaktflächen mit der Prozeßflüssigkeit haben muß, um die das Modul passierende Biomasse mechanisch nicht zu schädigen. Die Größe des Moduls richtet sich nach der zum Abtrennen einer entsprechenden Ammoniummenge erforderlichen Membranfläche. Diese errechnet sich aus den Stoffübergangsdaten.

### 2) Donorkreislauf:

Der Donorkreislauf wird als Bypaß um das Fermentationssystem angelegt, d.h. im Donorkreislauf zirkuliert die Fermentationsflüssigkeit, die nach Passieren des Moduls in das Fermentationssystem zurückgeführt wird. Als Umlaufpumpe sollte eine scherstreßarme Schlauchpumpe mit autoklavierbarem Pumpschlauch dienen, um dem direkten Anschluß an das biologische System Rechnung zu tragen.

### 3) Akzeptorkreislauf:

Der Kreislauf wird auf mindestens eine pH-Einheit höher als die Fermentation pH-statisiert und enthält eine Vorrichtung zum Entfernen von NH₃ aus der Akzeptorlösung. Diese Vorrichtung kann zum einen aus einem gerührten, druckstabilen und thermostatisierbaren Reaktionsgefäß bestehen. Hierin wird das NH₃ entweder durch Einleiten von sterilfiltriertem Helium oder durch ebenfalls steriles Anlegen eines Unterdrucks entfernt. Zum anderen kann die Vorrichtung aus einem zweiten Dialysemodul - wahlweise als Hollowfiber- oder Flachmembran - bestehen, das die Akzeptorlösung mit Hilfe einer mikroporösen Membran von einem Sekundärstrom trennt. Der Sekundärstrom wird auf pH-Werte kleiner 7 eingestellt, um eine Protonierung des NH₃ und somit einen ständigen Ammoniakgradienten über die poröse Membran zu gewährleisten.

## Patentansprüche

1. Verfahren zur Abtrennung von Ammonium aus kationenhaltigen wäßrigen Flüssigkeiten, insbesondere Fermentationsbrühen, über eine Transportmembran und selektive tive Elimination auf der Akzeptorseite,
**dadurch gekennzeichnet,**
daß man als Membran eine Kationenaustauschermembran verwendet und akzeptorseitig einen gegenüber der Donorseite derart erhöhten pH-Wert vorsieht, daß quer zur Membran ein NH₄⁺-Konzentrationsgefälle aufgrund des Gleichgewichts zwischen Ammonium und Ammoniak vorliegt, welch letzteres aus der akzeptorseitigen Lösung entfernt wird, deren Gehalt an von NH₄⁺ verschiedenen Kationen demjenigen der donorseitigen Lösung entspricht und/oder deren am Austausch über die Membran beteiligtes Volumen höchstens 10 % des donorseitigen Gesamtflüssigkeitsvolumens ausmacht.

2. Verfahren nach Anspruch 1,
**dadurch gekennzeichnet,**
daß die Entfernung des Ammoniaks aus der akzeptorseitigen Lösung durch Unterdruck und/oder Hindurchleiten von Inertgas oder durch selektiven Transport durch eine mikroporöse hydrophobe Membran in eine saure Protonierungslösung erfolgt.

3. Verfahren nach Anspruch 1 oder 2,
**dadurch gekennzeichnet,**
daß die donorseitige Flüssigkeit unmittelbar von einer pH-statischen Fermentation stammt.

4. Verfahren nach einem der vorangehenden Ansprüche,
**dadurch gekennzeichnet,**
daß akzeptorseitig eine pH-Erhöhung um zumindest eine pH-Einheit vorgesehen wird.

5. Verfahren nach einem der vorangehenden Ansprüche,
**dadurch gekennzeichnet,**
daß akzeptorseitig zellfreie Kulturflüssigkeit mit entsprechend verändertem pH-Wert vorgesehen wird.

6. Verfahren nach einem der vorangehenden Ansprüche,
**dadurch gekennzeichnet,**
daß akzeptorseitig ein Flüssigkeitsstrom von geringer Schichtdicke vorgesehen wird.

7. Verfahren nach einem der vorangehenden Ansprüche,
**gekennzeichnet durch**
ein Austauschvolumenverhältnis über die Membran hinweg zwischen Donor- und Akzeptorseite von ≧ 20.

8. Verfahren nach einem der vorangehenden Ansprüche,
**dadurch gekennzeichnet,**
daß als kationenhaltige Flüssigkeit Kulturflüssigkeit durch eine Zirkulationsleitung an der Donorseite der Kationenaustauschermembran entlanggeführt wird.

9. Vorrichtung zur Ammonium-Abtrennung aus kationenhaltiger wäßriger Flüssigkeit entsprechend einem der Ansprüche 1 bis 8 mit einem Behälter (2), der die kationenhaltige Flüssigkeit enthält und in eine Zirkulationsleitung (1) mit einer einkuppelbaren Durchflußstrecke (3) einbezogen ist,
**dadurch gekennzeichnet,**
daß die Durchflußstrecke (3) eine Kationenaustauschermembran (4) als eine Begrenzungswand aufweist, die gleichzeitig Begrenzungswand eines Akzeptorraumes (6') ist, der entweder unmittelbar Einrichtungen zur pH-Regelung und NH₃-Entfernung enthält oder Teil einer solche Einrichtungen (7-9) enthaltenden Zirkulationsleitung (7') ist.

## Claims

1. Process for separating ammonium from cation-containing aqueous liquids, in particular fermentation broths, by means of a transport membrane and selective elimination on the acceptor side, characterized in that the membrane used is a cation exchange membrane and, on the acceptor side, provision is made for an increased pH compared with the donor side such that an NH₄⁺ concentration gradient exists transverse to the membrane owing to the equilibrium between ammonium and ammonia, the latter of which is removed from the solution on the accepter side, whose content of cations other than NH₄⁺ corresponds to that of the solution on the donor side and/or whose volume involved in the exchange via the membrane makes up at most 10 % of the total liquid volume on the donor side.

2. Process according to Claim 1, characterized in that the removal of the ammonia from the solution on the acceptor side is effected by reduced pressure and/or passing through inert gas or by selective transport through a microporous hydrophobic membrane into an acidic protonation solution.

3. Process according to Claim 1 or 2, characterized in that the liquid on the donor side originates directly from a static-pH fermentation.

4. Process according to one of the preceding claims, characterized in that, on the acceptor side, provision is made for an increase in pH by at least one pH unit.

5. Process according to one of the preceding claims, characterized in that, on the acceptor side, provision is made for cell-free culture fluid having a correspondingly changed pH.

6. Process according to one of the preceding claims, characterized in that, on the acceptor side, provision is made for a liquid flow of low layer thickness.

7. Process according to one of the preceding claims, characterized by an exchange volume ratio across the membrane between the donor and acceptor side of ≧ 20.

8. Process according to one of the preceding claims, characterized in that, as cation-containing liquid, culture fluid is passed along by a circulation line on the donor side of the cation exchange membrane.

9. Device for the removal of ammonium from cation-containing aqueous liquid in accordance with one of Claims 1 to 8, having a container (2) which contains the cation-containing liquid and is included in a circulation line (1) having a couplable flow section (3), characterized in that the flow section (3) has a cation exchange membrane (4) as a limiting wall which is simultaneously a limiting wall of an acceptor space (6') which either directly contains devices for pH control and NH₃ removal or is part of a circulation line (7') containing such devices (7-9).

## Revendications

1. Procédé de séparation d'ammonium dans des liquides aqueux contenant des cations, en particulier des bouillons de fermentation, au moyen d'une membrane de transport et d'élimination sélective du côté accepteur, caractérisé en ce qu'on utilise, comme membrane, une membrane échangeuse de cations, et on prévoit, du côté accepteur, un pH qui, par rapport au côté donneur, est augmenté d'une manière telle qu'il y a, de part et d'autre de la membrane, une chute de la concentration de NH₄⁺ en raison de l'équilibre entre l'ammonium et l'ammoniac, ce dernier étant éliminé de la solution située du côté accepteur, dont la teneur en cations différents de NH₄⁺ correspond à celle de la solution située du côté donneur et/ou dont le volume prenant part à l'échange à travers la membrane représente au plus 10 % du volume total du liquide du côté donneur.

2. Procédé selon la revendication 1, caractérisé en ce qu'on effectue l'élimination de l'ammoniac dans la solution située du côté donneur en appliquant un vide et/ou en introduisant un gaz inerte ou grâce à un transport sélectif dans une solution acide de protonation, à travers une membrane hydrophobe poreuse.

3. Procédé selon la revendication 1 ou 2, caractérisé en ce que le liquide situé du côté donneur provient directement d'une fermentation à pH statique.

4. Procédé selon l'une des revendications précédentes, caractérisé en ce qu'il est prévu, du côté accepteur, une augmentation du pH d'au moins une unité de pH.

5. Procédé selon l'une des revendications précédentes, caractérisé en ce qu'il est prévu, du côté accepteur, un liquide de culture sans cellules, ayant un pH modifié d'une manière correspondante.

6. Procédé selon l'une des revendications précédentes, caractérisé en ce qu'il est prévu, du côté accepteur, un flux de liquide ayant une faible épaisseur de couche.

7. Procédé selon l'une des revendications précédentes, caractérisé par un rapport de volume d'échange de ≧ 20 à travers la membrane, entre le côté donneur et le côté accepteur.

8. Procédé selon l'une des revendications précédentes, caractérisé en ce que, comme liquide contenant des cations, un liquide de culture est amené par une canalisation de circulation le long du côté donneur de la membrane échangeuse de cations.

9. Dispositif pour la séparation de l'ammonium dans un liquide aqueux contenant des cations selon l'une des revendications 1 à 8, comportant un réservoir (2) qui contient le liquide contenant les cations et est inclus dans une canalisation de circulation (1) avec une trajectoire d'écoulement (3) raccordable, caractérisé en ce que la trajectoire d'écoulement (3) comporte une membrane échangeuse de cations, comme paroi de limitation, qui sert à la fois de paroi de limitation d'un compartiment acccepteur (6') qui contient directement des dispositifs pour le réglage du pH et l'élimination de NH₃ ou forme une partie d'une canalisation de circulation (7') contenant de tels dispositifs (7-9).
